# EUROPEAN PATENT APPLICATION

(11) **EP 1 352 974 A1**
(43) Date of publication of application: **15.10.2003**
(21) Application number: 03007393.6
(22) Date of filing: 02.04.2003
(51) Int. Cl.: C12Q 1/68

(54) **Oligonucleotide for detection of atypical mycobacteria mycobacterium avium and detection method**

(30) Priority: 02.04.2002 JP 2002099840
(71) Applicant: Tosoh Corporation, Shinnanyo-shi, Yamaguchi-ken, 746-8501 (JP)
(72) Inventor: Masuda, Noriyoshi, Tokyo (JP); Yasukawa, Kiyoshi, Kawasaki-shi, Kanagawa-ken (JP); Ishiguro, Takahiko, Yokohama-shi, Kanagawa-ken (JP)
(74) Representative: VOSSIUS & PARTNER

(57) **Abstract**

An oligonucleotide for detection of the Pst S-3 gene of an atypical mycobacterium Mycobacterium avium or an RNA derived from the gene consisting of at least 10 bases in any of SEQ ID NOS:1 to 12, which can bind to a specific site of the gene or the RNA.

SEQUENCE LISTING

<110> TOSOH corporation

<120> Oligonucleotide for detection of atypical mycobacteria mycobacterium avium
and detection method

<130> H 1609 EP

<160> 33

<210> 1
<211> 20
<212> DNA
<213> Artificial Sequence

<220>
<223> MAP-1R

<400> 1
ttccgcatcc cgacaacacc                                                    20

<210> 2
<211> 20
<212> DNA
<213> Artificial Sequence

<220>

<223> MAP－3R

<400> 2

gaatcgggtc atcgcgttgg                                                    20

<210> 3

<211> 23

<212> DNA

<213> Artificial Sequence

<220>

<223> MAP－4R

<400> 3

tgttcgaagg cgttgacgaa tcg                                                23

<210> 4

<211> 20

<212> DNA

<213> Artificial Sequence

<220>

<223> MAP－6R

<400> 4

gagtcggaac caccgaaatc                                                    20

<210> 5

<211> 20

<212> DNA

<213> Artificial Sequence

<220>

<223> MAP－7R

<400> 5

agattccagg ccggcgagcc 20

<210> 6

<211> 20

<212> DNA

<213> Artificial Sequence

<220>

<223> MAP－10R

<400> 6

tgcggaagac gacgtgaatc 20

<210> 7

<211> 20

<212> DNA

<213> Artificial Sequence

<220>

<223> MAP-11R

<400> 7

tatttctgga aattgtcggt 20

<210> 8

<211> 20

<212> DNA

<213> Artificial Sequence

<220>

<223> MAP-15R

<400> 8

tgttcagctt ctgcgcctga 20

<210> 9

<211> 20

<212> DNA

<213> Artificial Sequence

<220>

<223> MAP-16R

<400> 9

ctgatcgcca ccgcgtccgg                                                    20

<210> 10

<211> 20

<212> DNA

<213> Artificial Sequence

<220>

<223> MAP-17R

<400> 10

tcgagcacca gatcgttgcc                                                    20

<210> 11

<211> 20

<212> DNA

<213> Artificial Sequence

<220>

<223> MAP-19R

<400> 11

tgggggtcgg ggtacttcga                                                    20

<210> 12

<211> 22

<212> DNA

<213> Artificial Sequence

<220>
<223> MAP-20R

<400> 12
ttgaaggcgt cggggatcgg ga                                                22

<210> 13
<211> 23
<212> DNA
<213> Artificial Sequence

<220> 23
<223> MAP-3

<400> 13
cgcttcgtca acgccttcga aca                                               23

<210> 14
<211> 20
<212> DNA
<213> Artificial Sequence

<220>
<223> YO-avium-S-G

<400> 14

ttgccgttga attcgctgat                                                20

<210> 15

<211> 1264

<212> RNA

<213> Mycobacterium avium

<220>

<223> Standard RNA derived from the Pst S-3 gene of an atypical mycobacterium

Mycobacterium avium

<400> 15

gggagaacug uuagcuucgu ggugcgagac gggcuggucg gcacgcgguu gccgguggac  60

cggcggcgaa cauguuucau cccuaccgug aggaagcgaa uugaaacuca accgauuugg  120

ugccgugcug agcgucuuga gcgccggcgc gcugguguug ucgggaugcg gaagcgauaa  180

caacggggcc ggcgccggcg ccggcggcuc gucgucgagc aaggugagcu gcgguggcaa  240

gaaggcccuc aaggccagcg gcucgaccgc ccaggccaac gcgaugaccc gcuucgucaa  300

cgccuucgaa caggccuguc ccggccagac acugaacuac acggccaacg guuccggugc  360

cgggaucagc gaauucaacg gcaagcaaac cgauuucggu gguuccgacu cgccgcuggc  420

gcccagugaa uacgccgcgg cgcagcagcg uugcggcucg ccggccugga aucugccggu  490

gguuuucggc ccgaucgcca ucaccuacaa cgucgccggg cugaacucgc ugaaccugga  540

cggcgcgacc gccgccaaaa ucuucaacgg cgccaucacg acguggaaug acccggggau  600

ccaggcgcuc aaccccggcg uugcccugcc cgccgagccg auucacgucg ucuuccgcaa  660

cgacgaaucc ggcaccaccg acaauuucca gaaauaucuc gaugccgccg ccgacggcgc  720

cuggggcaag ggcgccggca agaccuucaa ggguggcguc ggcgagggag ccaagggcaa  780

cgacggcacc ucagcggcga ucaaggccac cgaagggucc aucaccuaca acgagugguc  840

guucgcucag gcgcagaagc ugaacauggc caagaucauc acgucggccg guccggacgc 900

gguggcgauc agcgccgacu cggugggcaa gacgaucgcg ggagccaaga ucuccggcca 960

gggcaacgau cuggugcucg acacccuguc guucuacaag cccacccagg ccggcucgua 1020

cccgaucgug cuggccaccu acgagaucgu cugcucgaag uaccccgacc cccaggucgg 1080

cacggcgguc aaggcguucc ugcagagcac cgucggcgcc ggccagaacg gucuggccga 1140

caacggcuau aucccgaucc ccgacgccuu caagucgaga cugucugcug ccaucaacgc 1200

gaucaccuaa ccugaggugg ucgugacacg aggaacagca accgcgggua ccgagcucga 1260

auuc 1264

<210> 16

<211> 20

<212> DNA

<213> Artificial Sequence

<220>

<223> MAP-2R

<400> 16

gggccttctt gccaccgcag 20

<210> 17

<211> 20

<212> DNA

<213> Artificial Sequence

<220>

<223> MAP-5R

<400> 17

gttggccgtg tagttcagtg                                                    20

<210> 18

<211> 20

<212> DNA

<213> Artificial Sequence

<220>

<223> MAP－8R

<400> 18

gttgtaggtg atggcgatcg                                                    20

<210> 19

<211> 20

<212> DNA

<213> Artificial Sequence

<220>

<223> MAP－9R

<400> 19

ttgagcgcct ggatccccgg                                                    20

<210> 20

<211> 20

<212> DNA

<213> Artificial Sequence

<220>

<223> MAP－12R

<400> 20

ttgccccagg cgccgtcggc                                                    20

<210> 21

<211> 20

<212> DNA

<213> Artificial Sequence

<220>

<223> MAP－13R

<400> 21

cgtcgttgcc cttggctccc                                                    20

<210> 22

<211> 20

<212> DNA

<213> Artificial Sequence

<220>

<223> MAP－14R

<400> 22
ttgtaggtga tggacccttc                                                    20

<210> 23
<211> 20
<212> DNA
<213> Artificial Sequence

<220>
<223> MAP－18R

<400> 23
tgggtgggct tgtagaacga                                                    20

<210> 24
<211> 24
<212> DNA
<213> Artificial Sequence

<220>
<223> MAP－3S

<400> 24
gaagcgggtc atcgcgttgg cctg                                               24

<210> 25

<211> 24

<212> DNA

<213> Artificial Sequence

<220>

<223> MAP-4S

<400> 25

tgttcgaagg cgttgacgaa gcgg 24

<210> 26

<211> 24

<212> DNA

<213> Artificial Sequence

<220>

<223> MAP-10S

<400> 26

tgcggaagac gacgtgaatc ggct 24

<210> 27

<211> 24

<212> DNA

<213> Artificial Sequence

<220>

<223> MAP-11S

<400> 27

tatttctgga aattgtcggt ggtg 24

<210> 28

<211> 24

<212> DNA

<213> Artificial Sequence

<220>

<223> MAP-15S

<400> 28

tgttcagctt ctgcgcctga gcga 24

<210> 29

<211> 51

<212> DNA

<213> Artificial Sequence

<220>

<223> MAP-3F

<400> 29

aattctaata cgactcacta tagggagacg cttcgtcaac gccttcgaac a 51

<210> 30

<211> 51

<212> DNA

<213> Artificial Sequence

<220>

<223> MAP-4F

<400> 30

aattctaata cgactcacta tagggagacg aacaggcctg tcccggccag a 51

<210> 31

<211> 51

<212> DNA

<213> Artificial Sequence

<220>

<223> MAP-10F

<400> 31

aattctaata cgactcacta tagggagatc cgcaacgacg aatccggcac c 51

<210> 32

<211> 51

<212> DNA

<213> Artificial Sequence

<220>

<223> MAP-11F

<400> 32

aattctaata cgactcacta tagggagaga aatatctcga tgccgccgcc g          51

<210> 33

<211> 51

<212> DNA

<213> Artificial Sequence

<220>

<223> MAP-15F

<400> 33

aattctaata cgactcacta tagggagatg aacatggcca agatcatcac g          51

## Description

The present invention relates to oligonucleotides and a method for detection of an atypical mycobacterium Mycobacterium avium in clinical tests. The oligonucleotides of the present invention are useful as reagents for gene diagnosis involving cleavage, amplification and detection of RNA or DNA or for inhibiting RNA reverse transcription or translation.

The atypical mycobacterium Mycobacterium avium (M. avium) falls into the same group as another atypical mycobacterium Mycobacterium intracellulare, called the MAC (Mycobacterium avium complex). The MAC causes pseudotuberculosis in patients with underlying pulmonary diseases and is responsible for about 70% of nontuberculous mycobacterial infections in Japan. MAC is usually resistant to various antibiotics.

Heretofore, identification of atypical mycobacteria including M. avium has been largely based on cumbersome incubation tests which take from 3 to 4 weeks for identification. Because not all strains of one species may show a property typical to the species, identification of atypical mycobacteria needs skills somewhat.

As discussed above, the test method in current use does not exactly meet such purposes as fast detection and verification of therapeutic effects required in practical medical settings. There are demands for development of a more sensitive and faster detection method which detects living bacteria only. Further, automated test instruments have been demanded to facilitate tests.

For high sensitivity, detection methods involving amplification of target nucleic acids is available. NASBA and 3SR are known as techniques for amplifying specific RNA sequences by the cooperative action of a reverse transcriptase and an RNA polymerase. These techniques involve the chain reaction comprising synthesis of a double-stranded DNA having a promoter sequence from the target RNA by using a primer having a promoter sequence, a reverse transcriptase and ribonuclease H, and formation by an RNA polymerase of an RNA having the specific base sequence, which is then used as the template for synthesis of the above-mentioned double-stranded DNA having the promoter sequence.

NASBA and 3SR allow isothermal amplification of nucleic acids and are considered suitable for automation. However, since the reactions involved in these amplification techniques are carried out at relatively low temperatures (for example, at 41°C), it is possible that the formation of an intramolecular structure of the target RNA lowers the reaction efficiency by hindering binding of the primers. Therefore, an operation of destroying the intramolecular structure of the target RNA such as heat denaturation of the target RNA are necessary before the amplification reaction to increase the binding efficiency of the primers. Oligonucleotides which can bind to RNAs having the above-mentioned intramolecular structure are also needed to detect RNA at low temperature.

The object of the present invention is to provide oligonucleotides useful for selective cleavage, amplification and sensitive detection and identification of an RNA derived from the Pst S-3 gene of Mycobacterium avium isothermally at relatively low temperatures (at 35°C to 50°C, preferably at 41°C) and preferable combinations of them.

The present invention has been accomplished to attain the above-mentioned object. The invention defined in Claim 1 of the present application provides an oligonucleotide needed for cleavage, detection or amplification of an RNA derived from the Pst S-3 gene of Mycobacterium avium, namely, an oligonucleotide consisting of at least 10 bases in any of SEQ ID NOS:1 to 12, which can specifically bind to the Pst S-3 gene of M. avium or an RNA derived from the gene.

The invention defined in Claim 2 of the present application provides the oligonucleotide according to Claim 1, which is an oligonucleotide probe which partly binds to the specific site of the RNA to cleave the RNA at the specific site. The invention defined in Claim 3 of the present application provides the oligonucleotide according to Claim 1, which is an oligonucleotide primer for DNA elongation. The invention defined in Claim 4 of the present application provides the oligonucleotide according to Claim 1, which is an oligonucleotide probe which is partly modified or labeled with a detectable label. The invention defined in Claim 5 of the present application provides the oligonucleotide according to Claim 1, which is a synthetic oligonucleotide containing a partial base substitution which does not impair the function of the oligonucleotide as an oligonucleotide probe.

The invention defined in Claim 6 of the present application provides an RNA amplification step for amplifying an RNA derived from the Pst S-3 gene of M. avium which comprises synthesizing a cDNA by the action of an RNA-dependent DNA polymerase by using a specific sequence in an RNA derived from the Pst S-3 gene of M. avium present in a sample as a template, degrading the RNA strand in the resulting RNA-DNA double strand by ribonuclease H to give a single-stranded DNA, forming a double-stranded DNA having a promoter sequence which can be transcribed into an RNA homologous or complementary to the specific sequence by using the single-stranded DNA as a template by the action of a DNA-dependent DNA polymerase, and then transcribing the double-stranded DNA into an RNA transcript, which acts as a template in the subsequent cDNA synthesis by the RNA-dependent DNA polymerase, wherein a first primer consisting of at least 10 consecutive bases in SEQ ID NO:13 which is homologous to the sequence of the RNA derived from the Pst S-3 gene of M. avium to be amplified and a second primer consisting of at least 10 consecutive bases in SEQ ID NO:4 which is complementary to part of the sequence of the RNA derived from the Pst S-3 gene of M. avium (either of which additionally has a promoter sequence for the RNA polymerase at the 5' end) are used.

The invention defined in Claim 7 of the present application provides the RNA detection step according to Claim 6, which comprises conducting the RNA amplification step in the presence of an oligonucleotide probe (having a sequence different from those of the first primer and the second primer) which can specifically bind to the RNA transcript resulting from the amplification and is labeled with an fluorescent intercalative dye, and measuring the change in the fluorescence from the reaction solution. The invention defined in Claim 8 of the present application provides the RNA detection step according to Claim 7, wherein the probe is designed to hybridize with at least part of the RNA transcript and alters its fluorescence upon hybridization. The invention defined in Claim 9 of the present application provides the RNA detection step according to Claim 8, wherein the probe has a sequence consisting of or complementary to at least 10 consecutive bases in SEQ ID NO:14.

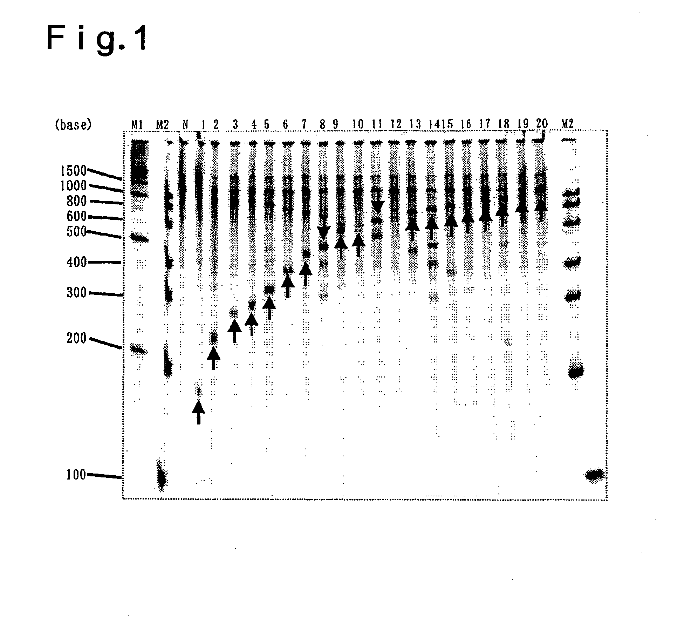

Fig. 1 is a (negative) photograph showing the results of urea-denatured 6% PAGE of samples after binding tests on oligonucleotides MAP-1R to MAP-20R for binding to a standard RNA derived from the Pst S-3 gene of Mycobacterium avium at 41°C. The specific bands are pointed by arrows. The results of the binding tests on MAP-1R to MAP-20R are shown in lanes 1 to 20. In lane N, the result obtained with the negative control (by using the diluent instead of an oligonucleotide) is shown. RNA Markers (0.1 to 1 kb and 0.2 to 10 kb) were used as molecular weight markers (lanes M1 and M2).

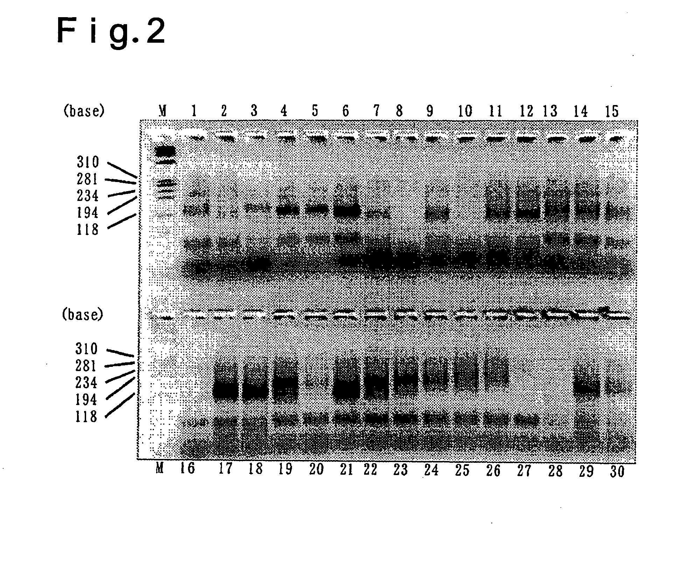

Fig. 2 is an electrophoretogram showing the results of RNA amplification from the initial RNA amount of 10³ copies/run in Example 2 using oligonucleotides in combinations (a) to (j) shown in Table 2. Lanes 1 and 2: combination (a). Lanes 4 and 5: combination (b). Lanes 7 and 8: combination (c). Lanes 10 and 11: combination (d). Lanes 13 and 14: combination (e). Lanes 16 and 17: combination (f). Lanes 19 and 20: combination (g). Lanes 22 and 23 combination (h). Lanes 25 and 26: combination (i). Lanes 28 and 29: combination (j). Lanes 3, 6, 9, 12, 18, 21, 24, 27 and 30: negative control (a diluent only instead of the RMA sample). The molecular weight marker, φX174/HaeIII digest (Marker 4) was used (lane M). Only combination (a) gave specific bands.

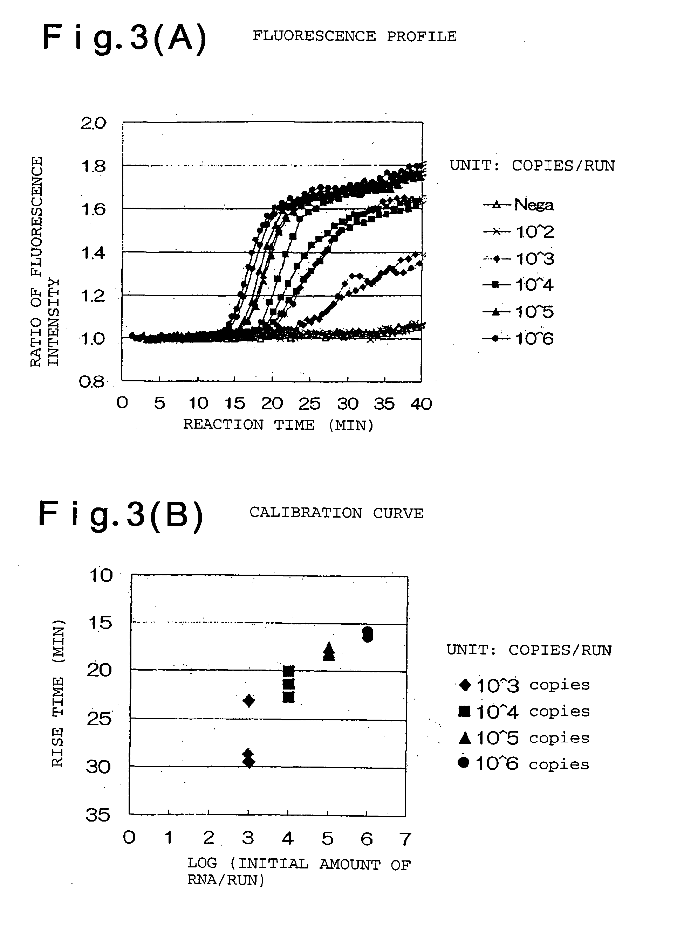

Fig. 3 is a graph (A) correlating the reaction time and the fluorescence enhancement accompanying RNA synthesis at initial RNA amounts of 10² copies/run to 10⁵ copies/run in Example 3 and a calibration curve (B) correlating the logarithm of the initial amount of the RNA and the rise time. It is demonstrated that it is possible to detect RNA at an initial amount of 10³ copies/run within 30 minutes and there is a correlation between the initial amount of the RNA and the rise time.

Now, the present invention will be described in detail.

The oligonucleotides of the present invention specifically hybridize with intramolecularly free regions in the target RNA during the above-mentioned RNA amplification and bind to the target RNA without heat denaturation as described previously. Thus, the present invention provides oligonucleotides which bind to intramolecularly free regions of an RNA derived from the Pst S-3 gene of M. avium isothermally at relatively low temperatures (from 35°C to 50°C, preferably 41°C) and is useful for selectively cleavage, amplification or detection of an RNA derived from the Pst S-3 gene of M. avium. More specifically, the present invention provides oligonucleotides used as oligonucleotide probes for cleavage of the target RNA at a specific site, as oligonucleotide primers for amplification of the target DNA by PCR, as oligonucleotide primers for amplification of the target RNA by NASBA or the like, or as oligonuclotide probes for detection of the target nucleic acid after or without such amplification, for successful fast and sensitive detection.

SEQ ID NOS:1 to 12 are embodiments of the oligonuleotides of the present invention useful for cleavage, amplification or detection of an RNA derived from the Pst S-3 gene of M. avium. The RNA derived from the Pst S-3 gene of M. avium includes RNAs obtained by using the gene as a template. Thought the oligonucleotides of the present invention may cover the full-length base sequences of SEQ ID NOS:1 to 12, they may be oligonucleotides consisting of at least 10 consecutive bases in the above-mentioned base sequences because about 10 bases are enough to secure the specific binding to the RNA derived from the Pst S-3 gene of M. avium.

The oligonucleotides of the present invention can, for example, be used as scissor probes for RNA cleavage. It is possible to cleave a target RNA at a specific site by hybridizing an oligonucleotide of the present invention to the target single-stranded RNA and treating the hybrid with an enzyme which cleaves the RNA in an RNA-DNA heteroduplex. As such an enzyme, an enzyme known to have a ribonuclease H activity may usually be used.

The oligonucleotides of the present invention can, for example, be used as oligonucleotide primers for amplification of nucleic acid. The use of the oligonucleotides of the present invention as primers in nucleic acid amplification enables only a nucleic acid derived from the Pst S-3 gene of M. avium as the target nucleic acid to be amplified. The amplification method may, for example, be PCR, LCR, NASBA or 3SR, but among them, an isothermal amplification method such as LCR, NASBA or 3SR is preferred. It is possible to detect M. avium by detecting the amplification product by various methods. For this purpose, one of the above-mentioned oligonucleotides other than those used for the amplification may be used as a probe, or the presence of the amplified fragment having the specific sequence may be confirmed by electrophoresis.

The oligonucleotides of the present invention may be used as probes, for example, after partly modified or labeled with a detectable label. In the case of detection of a target nucleic acid, hybridization of an oligonucleotide of the present invention labeled with a detectable label with a single-stranded target nucleic acid is followed by detection of the label on the hybridized probe. For the detection of the label, any method appropriate for the label may be used. For example, when a fluorescent intercalative dye is used to label an oligonucleotide, the use of a dye which shows an fluorescence enhancement upon intercalation into a nucleic acid double strand comprising the target nucleic acid and the oligonucleotide probe makes it easy to detect the hybridized probe only without removal of the probe unhybridized with the target nucleic acid. When an ordinary fluorescent dye is used as the label, detection may be preceded by removal of the probe unhybridiezed with the target nucleic acid. Before detection, it is preferred to amplify the target nucleic acid in a sample to a detectable amount by various nucleic acid amplification methods such as PCR, NASBA or 3SR, preferably by an isothermal nucleic acid amplification method such as NASBA or 3SR. On the other hand, when the labeled oligonucleotide probe is present in the reaction solution during amplification, it is particularly preferred to modify the probe, for example, by adding glycol acid to the 3' end so that the probe does not function as a nucleotide primer.

The present invention also provides a nucleic acid amplification step for amplifying an RNA derived from the Pst S-3 gene of M. avium in a sample or a method of detecting the RNA transcript obtained in the nucleic acid amplification step. The nucleic acid amplification step in the present invention includes PCR, NASBA and 3SR. Especially, an isothermal nucleic acid amplification method such as NASBA or 3SR which allows a specific RNA sequence derived from the Pst S-3 gene of M. avium to be amplified by the cooperative action of a reverse transcriptase and an RNA polymerase (under such conditions that the reverse transcriptase and the RNA polymerase act cooperatively).

For example, NASBA is an RNA amplification step comprising synthesizing a cDNA by the action of an RNA-dependent DNA polymerase by using a specific sequence in an RNA derived from the Pst S-3 gene of M. avium present in a sample as a template, degrading the RNA strand in the resulting RNA-DNA double strand by ribonuclease H to give a single-stranded DNA, forming a double-stranded DNA having a promoter sequence which can be transcribed into an RNA homologous or complementary to the specific sequence by using the single-stranded DNA as a template by the action of a DNA-dependent DNA polymerase, and then transcribing the double-stranded DNA into an RNA transcript, which acts as a template in the subsequent cDNA synthesis by the RNA-dependent DNA polymerase. The present invention is characterized by the use of a first primer consisting of at least 10 consecutive bases in SEQ ID NO:13 which is homologous to part of the sequence of the RNA derived from the Pst S-3 gene of M. avium and a second primer consisting of at least 10 consecutive bases in SEQ ID NO:4 which is complementary to part of the sequence of the RNA derived from the Pst S-3 gene of M. avium to be amplified (either of which additionally has a promoter sequence for the RNA polymerase at the 5' end).

Though the RNA-dependent DNA polymerase, the DNA-dependent DNA polymerase and the ribonuclease H are not particularly limited, but AMV reverse transcriptase is preferable because it has the activities of all of them. As the RNA polymerase, T7 phage RNA polymerase or SP6 phage RNA polymerase is preferred, though there is no particular restriction.

In the above-mentioned amplification step, even if the specific sequence is not present at the 5' end of the RNA, the RNA can be amplified by adding an oligonucleotide complementary to a region of the RNA derived from the Pst S-3 gene of M. avium which flanks the 5' end of the specific sequence with an overlap (of from 1 to 10 bases) with the specific sequence to cleave the RNA derived from the Pst S-3 gene of M. avium at the 5' end (by the action of a ribonuclease H) before it is used as the template in the initial stage of the nucleic acid amplification. For the cleavage, an oligonucleotide of any of SEQ ID NOS:1 to 12 (which is not used as the second primer in the above-mentioned amplification step) may be used. The scissor oligonucleotide for the cleavage is preferred to have a chemically modified hydroxyl group (for example, an aminated hydroxyl group) at the 3' end so as not to elongate from the 3' end.

Detection of the amplification product obtained in the nucleic acid amplification step is preferably carried out by measuring the change in the fluorescence of the reaction solution during the amplification step in the presence of an oligonucleotide labeled with a fluorescent intercalative dye, though it can be detected by conventional methods for detection of nucleic acid. The oligonucleotide may be, for example, an oligonucleotide having a fluorescent intercalative dye linked to a phosphorus atom in it via a linker. Such an oligonucleotide alters its fluorescence upon formation of a double strand with the target nucleic acid (a complementary nucleic acid) through intercalation of the intercalator moiety into the double strand and is characterized in that it obviates the need for separation analysis (Ishiguro, T. et al., (1996) Nucleic Acids Res. 24 (24) 4992-4997).

The sequence of the oligonucleotide is not particularly limited as long as it contains a sequence complementary to at least part of the amplification product, but it is preferably a sequence consisting of or complementary at least 10 consecutive bases in SEQ ID NO:14. It is also preferred to chemically modify the hydroxyl group at the 3' end of the oligonucleotide (for example, by adding glycolic acid) to prevent elongation reaction using the oligonucleotide as a primer.

Thus, amplification and detection of an RNA having the same sequence as a specific sequence in an RNA derived from the Pst S-3 gene of M. avium can be carried out in one tube at constant temperature in one step and can be automated easily.

Now, the present invention will be described in further detail by referring to Examples. However, the present invention is by no means restricted to these specific Examples.

### EXAMPLE 1

Oligonucleotides which specifically bind to an RNA derived from the Pst S-3 gene of an atypical mycobacterium Mycobacterium avium at 41°C were selected.

(1) A standard RNA (1264 bases, SEQ ID NO:15) which is an RNA derived from the Pst S-3 gene in the base sequence of M. avium was quantified by UV absorptiometry at 260 nm and diluted to 0.844 pmol/µL with an RNA diluent (10 mM Tris-HCl (pH 8.0), 0.1 mM EDTA, 1 mM DTT, 0.5 U/µl RNase inhibitor (Takara Shuzo Co., Ltd.)).

(2) 14.0 µL portions of a reaction solution of the following composition were dispensed into PCR tubes (capacity 0.5 ml: Gene Amp Thin-Walled Reaction Tubes, Perkin Elmer).
The composition of the reaction solution (in terms of the concentrations in the final volume of 15 µL)

| | |
|---|---|
| 60 mM | Tris-HCl buffer (pH 8.6) |
| 17 mM | Magnesium chloride |
| 90 mM | Potassium chloride |
| 6 U | RNase Inhibitor (Takara Shuzo Co., Ltd.) |
| 1 mM | DTT |
| 0.066 µM | standard RNA |
| 0.2 µM | oligonucleotide (the following oligonucleotides were used separately. The bases are numbered so that the base (base 101 in SEQ ID NO:15) at the starting point of the Pst S-3 region gene of M. avium is 1) |

MAP-1R (oligonucleotide complementary to bases 54 to 73 of the Pst S-3 gene, SEQ ID NO:1)
MAP-2R (oligonucleotide complementary to bases 129 to 148 of the Pst S-3 gene, SEQ ID NO:16)
MAP-3R (oligonucleotide complementary to bases 176 to 195 of the Pst S-3 gene, SEQ ID NO:2)
MAP-4R (oligonucleotide complementary to bases 190 to 212 of the Pst S-3 gene, SEQ ID NO:3)
MAP-5R (oligonucleotide complementary to bases 230 to 249 of the Pst S-3 gene, SEQ ID NO:17)
MAP-6R (oligonucleotide complementary to bases 292 to 311 of the Pst S-3 gene, SEQ ID NO:4)
MAP-7R (oligonucleotide complementary to bases 355 to 374 of the Pst S-3 gene, SEQ ID NO:5)
MAP-8R (oligonucleotide complementary to bases 392 to 411 of the Pst S-3 gene, SEQ ID NO:18)
MAP-9R (oligonucleotide complementary to bases 493 to 512 of the Pst S-3 gene, SEQ ID NO:19)
MAP-10R (oligonucleotide complementary to bases 540 to 559 of the Pst S-3 gene, SEQ ID NO:6)
MAP-11R (oligonucleotide complementary to bases 577 to 596 of the Pst S-3 gene, SEQ ID NO:7)
MAP-12R (oligonucleotide complementary to bases 610 to 629 of the Pst S-3 gene, SEQ ID NO:20)
MAP-13R (oligonucleotide complementary to bases 666 to 685 of the Pst S-3 gene, SEQ ID NO:21)
MAP-14R (oligonucleotide complementary to bases 712 to 731 of the Pst S-3 gene, SEQ ID NO:22)
MAP-15R (oligonucleotide complementary to bases 747 to 766 of the Pst S-3 gene, SEQ ID NO:8)
MAP-16R (oligonucleotide complementary to bases 793 to 812 of the Pst S-3 gene, SEQ ID NO:9)
MAP-17R (oligonucleotide complementary to bases 862 to 881 of the Pst S-3 gene, SEQ ID NO:10)
MAP-18R (oligonucleotide complementary to bases 889 to 908 of the Pst S-3 gene, SEQ ID NO:23) .
MAP-19R (oligonucleotide complementary to bases 955 to 974 of the Pst S-3 gene, SEQ ID NO:11)
MAP-20R (oligonucleotide complementary to bases 1052 to 1073 of the Pst S-3 gene, SEQ ID NO:12)

### Distilled water for volume adjustment

(3) The reaction solutions were incubated at 41°C for 5 minutes, and 1 µL of a 8U/µL AMV reverse transcriptase solution (Takara Shuzo Co., Ltd.) (which cleaves the RNA strand in an DNA/RNA double strand) was added.

(4) Then, the PCR tubes were incubated at 41°C for 10 minutes again.

(5) The cleavage fragments obtained by the reaction were identified by urea-denatured polyacrylamide gel electrophoresis (polyacrylamide concentration 6%, urea 7 M) followed by staining with SYBR Green II (Takara Shuzo Co., Ltd.). The cleavage of the target RNA by the ribonuclease H activity of AMV reverse transcriptase on the RNA strand in the DNA/RNA double strand formed by hybridization of an oligonucleotide with a specific region of the target RNA can be confirmed by the appearance of specific bands.

The results (negative photograph) of the electrophoresis are shown in Fig. 1. The regions of the target RNA which formed hybrids with oligonucleotides were degraded to give degradation products of specific lengths. Table 1 shows the sites of the target RNA to which the respective oligonucleotides are supposed to bind specifically and the lengths of the bands which are supposed to be formed. In the cases of MAP-1R, MAP-3R, MAP-4R, MAP-R6, MAP-7R, MAP-10R, MAP-11R, MAP-15R, MAP-16R, MAP-17R, MAP-19R and MAP-20R, specific bands which indicate strong binding of these oligonucleotides to the RNA derived from the Pst S-3 gene of M. avium at a constant temperature of 41°C were observed. The bases in Table 1 are numbered so that the base (base 101 in SEQ ID NO:15) at the starting point of the Pst S-3 region gene of M. avium is 1. In the table, ○ indicates that only specific bands were observed, Δ indicates that both specific bands and non-specific bands were observed, and × indicates that no specific bands were observed.

**Table 1**

| Oligonucleotide | Location of the base | Length of supposed band (base) | Results |
|---|---|---|---|
| MAP-1R | 54 | 153, 1085 | ○ |
| MAP-2R | 129 | 228, 1010 | Δ |
| MAP-3R | 176 | 275, 963 | ○ |
| MAP-4R | 190 | 289, 946 | ○ |
| MAP-5R | 230 | 329, 909 | Δ |
| MAP-6R | 292 | 391, 847 | ○ |
| MAP-7R | 355 | 454, 784 | ○ |
| MAP-8R | 392 | 491, 747 | Δ |
| MAP-9R | 493 | 592, 646 | Δ |
| MAP-10R | 540 | 639, 599 | ○ |
| MAP-11R | 577 | 676, 562 | ○ |
| MAP-12R | 610 | 709, 529 | × |
| MAP-13R | 666 | 765, 473 | Δ |
| MAP-14R | 712 | 811, 427 | Δ |
| MAP-15R | 747 | 846, 392 | ○ |
| MAP-16R | 793 | 892, 346 | ○ |
| MAP-17R | 862 | 961, 277 | ○ |
| MAP-18R | 889 | 988, 250 | Δ |
| MAP-19R | 955 | 1054, 184 | ○ |
| MAP-20R | 1052 | 1151, 85 | ○ |

### EXAMPLE 2

RNA amplification was carried out using oligonucleotides which bind specifically to an RNA derived from the Pst S-3 gene of M. avium.

(1) The same standard RNA derived from the Pst S-3 gene of M. avium as used in Example 1 was quantified by UV absorptiometry at 260 nm and diluted to 10³ copies/5 µL with an RNA diluent (10 mM Tris-HCl (pH 8.0), 1 mM EDTA, 1 mM DTT, 0.5 U/µL RNase inhibitor (Takara Shuzo Co., Ltd.)). The diluent alone was used as a control sample (Nega).

(2) 20 µL portions of a reaction solution of the following composition were dispensed into 0.5 mL PCR tubes (Gene Amp Thin-Walled Reaction Tubes, Perkin Elmer), and 5 µL of the RNA sample was added.
The composition of the reaction solution (in terms of the concentrations in the final volume of 30 µL)

| | |
|---|---|
| 60 mM | Tris-HCl buffer (pH 8.6) |
| 17 mM | Magnesium chloride |
| 150 mM | Potassium chloride |
| 6 U | RNase Inhibitor |
| 1 mM | DTT |
| 0.25 mM | each of dATP, dCTP, dGTP and dTTP |
| 3.6 mM | ITP |
| 3.0 mM | each of ATP, CTP, GTP and UTP |
| 0.16 µM | First oligonucleotide |
| 1.0 µM | Second primer |
| 1.0 µM | Third primer |
| 13% | DMSO |

### Distilled water for volume adjustment

(3) RNA amplification was carried out in the solutions which contained the combinations of a first oligonucleotide, a second oligonucleotide and a third oligonucleotide shown in Table 2.

(4) The reaction solutions were incubated at 41°C for 5 minutes, and 5 µL of an enzyme solution of the following composition was added.
The composition of the enzyme solution (in terms of the concentrations in the final volume of 30 µL)

| | |
|---|---|
| 2.0% | Sorbitol |
| 3.6 µg | Bovine serum albumin |
| 142 U | T7 RNA polymerase (GIBCO) |
| 8 U | AMV reverse transcriptase (Takara Shuzo Co., Ltd.) |

### Distilled water for volume adjustment

(5) The PCR tubes were incubated at 41°C for 30 minutes again.

(6) The regions of the RNA amplified in the reaction were identified by agarose gel electrophoresis (agarose concentration 4%) followed by staining with SYBR Green II (Takara Shuzo Co., Ltd.). The RNA segments between the second and third oligonucleotides were amplified subsequent to the binding of oligonucleotide probes to specific regions of the target RNA and detected as specific bands.

The results (negative photograph) of the electrophoresis are shown in Fig. 2. The lengths of the products of the amplification reaction observed as specific bands are shown in Table 2. Among the combinations of oligonucleotides shown in Table 2, combination (a) gave specific bands after the RNA amplification reaction and proved to be useful for detection of M. avium.

**Table 2**

| Combination | First oligo-nucleotide | Second oligo-nucleotide | Third oligo-nucleotide | Length of amplification product (bases) |
|---|---|---|---|---|
| (a) | MAP-3S | MAP-3F | MAP-6R | 128 |
| (b) | MAP-3S | MAP-3F | MAP-7R | 191 |
| (c) | MAP-4S | MAP-4F | MAP-6R | 111 |
| (d) | MAP-4S | MAP-4F | MAP-7R | 174 |
| (e) | MAP-10S | MAP-10F | MAP-15R | 219 |
| (f) | MAP-10S | MAP-10F | MAP-16R | 265 |
| (g) | MAP-10S | MAP-10F | MAP-17R | 334 |
| (h) | MAP-11S | MAP-11F | MAP-15R | 182 |
| (i) | MAP-11S | MAP-11F | MAP-17R | 297 |
| (j) | MAP-15S | MAP-15F | MAP-17R | 141 |

Table 2 shows the combinations of the first, second and third oligonucleotides used in the present experiment system and the lengths of the specific bands detected as the products of the RNA amplification using them. The first oligonucleotides had aminated hydroxyl groups at the 3' end. The second oligonucleotides had base sequences (SEQ ID NO: 30) which additionally had the T7 promoter region at the 5' end from "A" at position 1 from the 5' end to "A" at position 22 and the subsequent enhancer sequence from "G" at position 23 to "A" at position 28. The bases are numbered so that the base at the starting point of the Pst S-3 gene in SEQ ID NO:15 is 1.
The first oligonucleotides
   MAP-3S (SEQ ID NO:24, from base 172 to base 195)
   MAP-4S (SEQ ID NO:25, from base 189 to base 212)
   MAP-10S (SEQ ID NO:26, from base 537 to base 559)
   MAP-11S (SEQ ID NO:27, from base 573 to base 596)
   MAP-15S (SEQ ID NO:28, from base 743 to base 766)
The second oligonucleotides
   MAP-3F (SEQ ID NO:29, from base 190 to base 207)
   MAP-4F (SEQ ID NO:30, from base 207 to base 229)
   MAP-10F (SEQ ID NO:31, from base 554 to base 576)
   MAP-11F (SEQ ID NO:32, from base 591 to base 613)
   MAP-15F (SEQ ID NO:33, from base 761 to base 783)
The third oligonucleotides
   MAP-6R (SEQ ID NO:4, from base 292 to base 311)
   MAP-7R (SEQ ID NO:5, from base 355 to base 374)
   MAP-15R (SEQ ID NO:8, from base 747 to base 766)
   MAP-17R (SEQ ID NO:10, from base 862 to base 881)

### EXAMPLE 3

The target RNA derived from the Pst S-3 gene of M. avium at various initial copy numbers was detected using the combinations of oligonucleotides of the present invention.

(1) The same standard RNA derived from the Pst S-3 gene of M. avium as used in Example 1 was diluted to 10⁶-10² copies/5 µL with an RNA diluent (10 mM Tris-HCl (pH 8.0), 1 mM EDTA, 0.5 U/µL RNase inhibitor (Takara Shuzo Co., Ltd.), 5 mM DTT). The diluent alone was used as a control sample (negative).

(2) 20 µL portions of a reaction solution of the following composition were dispensed into 0.5 mL PCR tubes (Gene Amp Thin-Walled Reaction Tubes, Perkin Elmer), and 5 µL of the RNA sample was added. The composition of the reaction solution (in terms of the concentrations in the final volume of 30 µL)

| | |
|---|---|
| 60 mM | Tris-HCl buffer (pH 8.6) |
| 17 mM | Magnesium chloride |
| 120 mM | Potassium chloride |
| 6 U | RNase Inhibitor |
| 1 mM | DTT |
| 0.25 mM | each of dATP, dCTP, dGTP and dTTP |
| 3.6 mM | ITP |
| 3.0 mM | each of ATP, CTP, GTP and UTP |
| 0.16 µM | First oligonucleotide (MAP-3S, SEQ ID NO:24, having an aminated hydroxyl group at the 3' end) |
| 1.0 µM | Second oligonucleotide (MAP-3F, SEQ ID NO:29) |
| 1.0 µM | Third oligonucleotide (MAP-6R, SEQ ID NO:4) |
| 25 nM | Oligonucleotide labeled with a fluorescent intercalative dye (YO-avium-S-G, SEQ ID NO:14, having the fluorescent intercalative dye between "T" at position 7 from the 5' end and "T" at position 8 and having a hydroxyl group modified with glycolic acid at the 3' end) |
| 13% | DMSO |

### Distilled water for volume adjustment

(3) The reaction solutions were incubated at 41°C for 5 minutes, and 5 µL of an enzyme solution of the following composition which was pre-incubated at 41°C for 2 minutes was added.
The composition of the enzyme solution (in terms of the concentrations in the final volume of 30 µL)

| | |
|---|---|
| 2.0% | Sorbitol |
| 3.6 µg | Bovine serum albumin |
| 142 U | T7 RNA polymerase (GIBCO) |
| 8 U | AMV reverse transcriptase (Takara Shuzo Co., Ltd.) |

### Distilled water for volume adjustment

(4) The fluorescence intensities of the reaction solutions in the PCR tubes were directly monitored at 41°C in a thermostatic fluorescent spectrophotometer at an excitation wavelength of 470 nm and an emission wavelength of 510 nm.

The time courses of the ratio of fluorescence intensities of the samples (fluorescence intensity at a certain time/background fluorescence intensity) from addition of the enzyme solution at 0 minute were shown in Fig. 3(A). The relation between the logarithm of the initial amount of the RNA and the rise time required until the ratio of fluorescence intensity reached the negative average plus triple the standard deviation:1.2) is shown in Fig. 3(B). The initial amounts of the RNA were from 10² copies/run to 10⁶ copies/run.

Fig. 3 demonstrates that it was possible to detect 10³ copies within about 30 minutes. The fluorescence profiles dependent on the initial concentrations of the standard RNA suggest that it is possible to quantify the RNA derived from the Pst S-3 region of M. avium in an unknown sample. Thus, it is proved that the present invention allows fast and sensitive detection of M. avium.

As described above, the oligonucleotides of the present invention are hybridizable with an RNA derived from the Pst S-3 gene of M. avium at relatively low and constant temperatures (from 35°C to 50°C, preferably 41°C) at which formation of an intramolecular structure of the RNA can occur to hinder binding of primers or probes. Therefore, the oligonucleotides can bind to the target RNA specifically without heat denaturation of the target RNA. Thus, the oligonucleotides of the present invention are useful as oligonucleotide primers or oligonucleotide probes used in RNA amplification for cleavage, amplification or detection of an RNA derived from the Pst S-3 gene of M. avium.

In addition to the above-mentioned purposes, the oligonucleotides of the present invention are also useful for amplification or detection of the Pst S-3 gene of M. avium.

The oligonucleotides of the present invention are not limited to those having the base sequences consisting of 20 bases to 23 bases in the Sequence Listing and may be oligonucleotides consisting of at least 10 consecutive bases in these sequences. It is obvious from the fact that a base sequence of about 10 bases is enough to secure the specific binding of a primer or a probe to a target nucleic acid at relatively low temperatures (preferably 41°C).

The entire disclosure of Japanese Patent Application No. 2002-099840 filed on April 2, 2002 including specification, claims, drawings and summary is incorporated herein by reference in its entirety.

## Claims

1. An oligonucleotide for detection of the Pst S-3 gene of an atypical mycobacterium Mycobacterium avium or an RNA derived from the gene consisting of at least 10 bases in any of SEQ ID NOS:1 to 12, which can bind to a specific site of the gene or the RNA.

2. The oligonucleotide according to Claim 1, which is an oligonucleotide probe which binds to the specific site of the RNA to cleave the RNA at the specific site.

3. The oligonucleotide according to Claim 1, which is an oligonucleotide primer for DNA elongation.

4. The oligonucleotide according to Claim 1, which is an oligonucleotide probe which is partly modified or labeled with a detectable label.

5. The oligonucleotide according to Claim 1, which is a synthetic oligonucleotide containing a partial base substitution which does not impair the function of the oligonucleotide as an oligonucleotide probe.

6. A detection method using an RNA amplification step for amplifying an RNA derived from the Pst S-3 gene of Mycobacterium avium which comprises synthesizing a cDNA by the action of an RNA-dependent DNA polymerase by using a specific sequence in an RNA derived from the Pst S-3 gene of an atypical mycobacterium Mycobacterium avium present in a sample as a template, degrading the RNA strand in the resulting RNA-DNA double strand by ribonuclease H to give a single-stranded DNA, forming a double-stranded DNA having a promoter sequence which can be transcribed into an RNA homologous or complementary to the specific sequence by using the single-stranded DNA as a template by the action of a DNA-dependent DNA polymerase, and then transcribing the double-stranded DNA into an RNA transcript, which acts as a template in the subsequent cDNA synthesis by the RNA-dependent DNA polymerase, wherein a first primer consisting of at least 10 consecutive bases in SEQ ID NO:13 which is homologous to part of the sequence of the RNA derived from the Pst S-3 gene of Mycobacterium avium to be amplified and a second primer consisting of at least 10 consecutive bases in SEQ ID NO:4 which is complementary to part of the sequence of the RNA derived from the Pst S-3 gene of Mycobacterium avium (either of which additionally has a promoter sequence for the RNA polymerase at the 5' end) are used.

7. The detection method according to Claim 6, which comprises conducting the RNA amplification step in the presence of an oligonucleotide probe (having a sequence different from those of the first primer and the second primer) which can specifically bind to the RNA transcript resulting from the amplification and is labeled with an fluorescent intercalative dye, and measuring the change in the fluorescence from the reaction solution.

8. The detection method according to Claim 7, wherein the probe is designed to hybridize with at least part of the RNA transcript and alters its fluorescence upon hybridization.

9. The detection method according to Claim 8, wherein the probe has a sequence consisting of or complementary to at least 10 consecutive bases in SEQ ID NO:14.
